Europäisches Patentamt

European Patent Office

Office européen des brevets

(19)

(11) Numéro de publication: **0 237 430**
**B1**

(12) **FASCICULE DE BREVET EUROPEEN**

(45) Date de publication du fascicule du brevet:
**12.12.90**

(51) Int. Cl.⁵: **C02F 3/28**

(21) Numéro de dépôt: **87400519.2**

(22) Date de dépôt: **10.03.87**

(54) Procédé pour fermentation méthanique à film fixe et à double flux.

(30) Priorité: **10.03.86 FR 8603373**

(43) Date de publication de la demande:
**16.09.87 Bulletin 87/38**

(45) Mention de la délivrance du brevet:
**12.12.90 Bulletin 90/50**

(84) Etats contractants désignés:
**BE CH DE ES GB IT LI NL**

(56) Documents cités:
**EP-A- 0 057 152**
**EP-A- 0 159 135**
**FR-A- 2 235 089**
**FR-A- 2 369 217**
**FR-A- 2 552 298**
**US-A- 4 336 135**

**Le dossier contient des informations techniques**
**présentées postérieurement au dépôt de la demande et**
**ne figurant pas dans le présent fascicule.**

(73) Titulaire: **SOCIETE GENERALE POUR LES TECHNIQUES**
**NOUVELLES S.G.N. Société anonyme dite:, 1, rue des**
**Hérons Montigny-le-Bretonneux,**
**F-78184 Saint-Quentin-en-Yvelines Cédex(FR)**

(72) Inventeur: **Camilleri, Claude, 26, Rue Vasco De Gama,**
**F-75015 Paris(FR)**

(74) Mandataire: **Le Roux, Martine et al, Cabinet Beau de**
**Loménie 55, rue d'Amsterdam, F-75008 Paris(FR)**

ACTORUM AG

**Description**

La présente invention concerne un procédé et un dispositif pour fermentation méthanique à film fixe et à double flux.

L'invention est applicable à une installation pour la fermentation anaérobie d'effluents contenant des matières organiques solubles ou en suspension.

Pour plus de clarté dans la suite du texte, le terme substrat désignera l'ensemble de l'effluent à traiter et des matières recyclées. Son débit est égal au débit de l'effluent à traiter plus le débit de recyclage.

A l'échelle industrielle, on connaît des procédés de traitement par fermentation anaérobie avec circulation du substrat à travers un lit à garnissage sur lequel se trouvent les micro-organismes actifs, le substrat circulant soit de bas en haut, soit du haut vers le bas.

Ainsi, le brevet FR-A-81/O1564 décrit la dégradation d'un substrat en circulation descendante à travers un lit à garnissage. Le recyclage d'une partie des boues permet d'augmenter la dégradation des matières en suspension, mais a l'inconvénient de produire un liquide épuré encore fortement chargé en matières en suspension (MES) qu'il faudra clarifier par décantation par exemple. Pour limiter cet inconvénient, dans le brevet précité, la sortie du liquide épuré se fait au niveau du garnissage par un déversoir. Pour améliorer encore la séparation solide-liquide, il faudrait abaisser le débit de l'effluent à l'entrée du fermenteur, donc diminuer sa vitesse d'écoulement. Mais, dans ce cas, la production en effluent traité à l'échelle industrielle n'est pas suffisante. La demanderesse a résolu ce problème par le procédé objet de l'invention.

Le brevet US-A 4 336 135 décrit un appareillage pour le traitement des effluents, qui est agencé de façon à permettre le traitement en deux étapes, l'une se déroulant dans une zone centrale à écoulement descendant et l'autre se déroulant dans une zone périphérique annulaire à écoulement ascendant.

Mais ce brevet est muet sur les conditions opératoires à respecter pour pouvoir traiter dans un appareillage de ce type des effluents ayant une certaine charge en matières en suspension et ayant une demande élevée en oxygène.

La présente invention s'applique à tout effluent ayant une teneur en matières en suspension (MES) supérieure ou égale à 1 g/l et ayant une demande chimique en oxygène (DCO) supérieure ou égale à 5 OOO mg/l.

Le procédé selon l'invention comportera donc les étapes suivantes :

Etape 1. Fermentation anaérobie du substrat en écoulement descendant à travers un lit à garnissage.

Etape 2. Décantation du mélange issu de l'étape 1 pour donner des boues et un liquide surnageant, avec recyclage dudit liquide et éventuellement d'une partie des boues.

Etape 3. Fermentation anaérobie du liquide surnageant issu de l'étape 2 à travers un lit à garnissage, ledit liquide traversant le lit avec un écoulement ascendant.

La description sera mieux suivie en se référant aux figures :

- la figure 1 représente schématiquement le dispositif utilisable pour la mise en oeuvre du procédé ;
- la figure 2 décrit un dispositif particulièrement intéressant pour la mise en oeuvre dudit procédé ;
- la figure 3 et la figure 4 montrent un fermenteur utilisable avec ledit procédé.

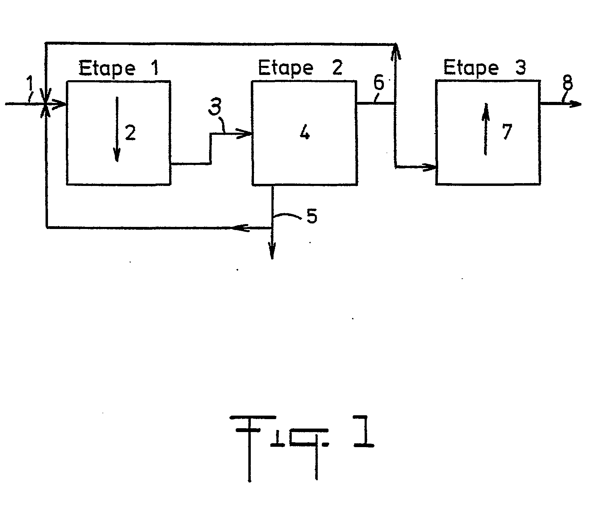

Le procédé objet de l'invention comprend les trois étapes ci-dessous décrites et se déroulant dans l'ordre indiqué (figure 1).

Etape 1 : fermentation anaérobie du substrat en écoulement descendant sur lit à garnissage.

Le substrat 1 est amené dans une zone de fermentation anaérobie 2, dans laquelle les micro-organismes actifs sont retenus dans un garnissage occupant cette zone. Le substrat 1 traverse cette zone en écoulement descendant, symbolisé par la flèche axée vers le bas sur la figure 1.

Le substrat est formé de l'effluent à traiter, du liquide recyclé et éventuellement des boues recyclées.

Le débit des matières recyclées (liquide + boues éventuellement) est supérieur au débit de l'effluent à traiter et, de préférence, compris entre 1 et 1O fois celui dudit effluent.

Dans le cas du recyclage de liquide et de boues, le débit du liquide assure près de 9O % du débit total.

Les boues peuvent être recyclées de façon intermittente.

Le recyclage des boues permet de réintroduire dans la zone de fermentation 2 des micro-organismes de façon à maintenir un potentiel de dégradation dans cette zone. Par ailleurs, la demanderesse a constaté que des micro-organismes différents de ceux présents dans la zone 2 se développent dans les boues décantées. Le recyclage d'une partie desdites boues permet alors d'introduire ces autres micro-organismes dans la zone 2 et, par conséquent, de favoriser encore la dégradation. Le liquide recyclé provient de la décantation de l'étape 2. Ce liquide, dont une partie des matières dégradables a été transformée en gaz, présente un pH supérieur à celui du substrat en cours de transformation en acide en biogaz dans la zone 2.

Le recyclage dudit liquide permet d'augmenter le rendement de la dégradation mais aussi de réguler le pH du substrat.

Tout type de garnissage convient. Il est néanmoins préféré des garnissages avec des anneaux ondulés, du type FLOCOR R par exemple, ou bien des plaques, du type FLOROR E, ou bien des houppes et des pompons par exemple.

Le mélange 3 sortant est envoyé à la deuxième étape du procédé.

Etape 2 : décantation.

Le mélange 3 issu de l'étape 1 subit une décantation dans une zone 4 dite zone de décantation.

La décantation peut être effectuée en une ou plusieurs étapes ; tout autre mode de séparation peut être adapté aux besoins.

De préférence, cette zone 4 coexiste avec la zone 2 dans le même fermenteur ; la description des dispositifs illustrera cette disposition avantageuse pour la mise en oeuvre du procédé, mais qui ne modifie pas les caractéristiques dudit procédé.

De cette étape, deux phases sont obtenues :
- une phase dite "solide" 5, appelée "boues",
- une phase liquide 6 : le liquide surnageant pouvant comprendre encore quelques MES non séparées et comprenant encore des matières solubles.

Une partie des boues 5 peut être recyclée à l'étape 1, une autre partie est purgée.

Une fraction du liquide 6 est recyclée à l'étape 1, une autre partie est envoyée à l'étape 3.

Etape 3 :

La phase liquide 6 en provenance de l'étape 2 contenant encore des matières dégradables est soumise à l'action de micro-organismes retenus par un garnissage et dans des conditions anaérobies, ce dans une zone de fermentation 7. La phase liquide 6 traverse cette zone 7 dans le sens ascendant, ainsi que l'indique la flèche sur la figure 1.

De même que pour la zone de fermentation 2, tout garnissage peut convenir. Le même garnissage pourra être choisi.

Les essais faits par la demanderesse ont montré que la vitesse de l'écoulement descendant est limitée par le garnissage choisi. Ainsi, par exemple,avec un garnissage d'anneaux FLOCOR R, on ne peut dépasser en écoulement descendant 2,5 m/h au risque de lessiver le milieu. De préférence, la vitesse descendante sera de l'ordre de 1,5 m/h. Avec un garnissage FLOCOR R, la vitesse de l'écoulement ascendant n'excède pas O,5 m/h, de préférence elle est comprise entre O,3 et O,5 m/h.

Il sort de la zone 7 un liquide 8 épuré dont la DCO a été réduite de l'ordre de 8O à 9O %.

L'invention peut donc être définie comme suit :
procédé de traitement biologique d'effluents contenant des matières organiques en suspension et/ou en solution par fermentation méthanique comprenant deux étapes successives de fermentation anaérobie à travers un lit à garnissage, la première en écoulement descendant, la seconde en écoulement ascendant, caractérisé en ce que les deux opérations sont séparées par une étape de décantation avec recyclage vers la première étape d'une partie du liquide surnageant obtenu et éventuellement d'une partie des boues décantées, que le débit dudit recyclage (liquide surnageant et éventuellement boues) est supérieur au débit de l'effluent à traiter et de préférence compris entre 1 et 1O fois celui dudit effluent, et que ledit procédé est appliqué à des effluents à traiter ayant une DCO $\geq$ 5 OOO mg/l et une teneur en MES $\geq$ 1 g/l.

Un tel procédé présente les avantages :

- de maintenir autant que possible les MES dans le système, donc de sortir un liquide relativement clair,
- de maintenir les micro-organimes dans le système, ce qui évite des séparations ultérieures et permet de conserver un potentiel de dégradation,
- d'augmenter le temps de séjour des MES dans le système afin de les valoriser au mieux, ce qui permet de traiter en fermentation anaérobie des effluents fortement chargés avec un bon rendement d'épuration sans sortir de MES ou de micro-organimes du système, et ce avec des débits suffisants pour une application à l'échelle industrielle.

L'invention peut être mise en oeuvre dans un dispositif comprenant :
- des moyens pour effectuer une fermentation anaérobie sur lit à garnissage d'un substrat en écoulement descendant,
- des moyens pour effectuer une décantation sur le mélange précédemment obtenu pour donner un liquide surnageant et des boues,
- des moyens pour recycler une partie du liquide surnageant et une partie des boues,
- des moyens pour effectuer une fermentation anaérobie sur lit à garnissage du liquide surnageant en écoulement ascendant,
- des moyens pour amener le substrat, soutirer le liquide épuré, purger les boues et transférer le mélange et le liquide surnageant.

Selon la figure 1, les moyens pour effectuer la fermentation anaérobie du substrat 1 selon l'étape 1 contiennent un lit à garnissage reposant sur une grille et constituant la zone de fermentation 2.

Le garnissage est composé de préférence d'anneaux ondulés en polychlorure de vinyle du type FLOCOR R ou bien de houppes et de pompons. D'autres types d'éléments peuvent tout aussi bien convenir : plaques, copeaux... en argile expansée, matière plastique, textile...

Le substrat est amené au-dessus dudit lit à garnissage, de préférence par arrosage dudit lit, de façon à assurer une bonne répartition du substrat.

Les moyens pour effectuer la décantation selon l'étape 2 en boues et liquide surnageant dans une zone de décantation 4 peuvent être indépendants des moyens de fermentation selon l'étape 1. Ce sont par exemple un décanteur, une centrifugeuse...

Les moyens pour effectuer la décantation peuvent aussi être agencés dans les moyens pour effectuer la fermentation selon l'étape 1, de façon que 1a zone 4 de décantation et la zone 2 de fermentation fassent partie du même dispositif. Ainsi, dans un dispositif dit fermenteur-décanteur, la zone de décantation 4 est située au-dessous de la zone de fermentation 2, le mélange obtenu par fermentation s'écoulant alors par gravité dans la zone 4.

La grille soutenant le lit à garnissage de la zone de fermentation 2 est placée à une hauteur h (comptée à partir du bas de la zone de décantation 4) telle que h/H O,1 à O,5 et, de préférence h/H = O,25 avec H = hauteur totale (zone 4 + zone 2).

Avantageusement, pour favoriser la décantation, la zone 4 présente la forme d'un cône ouvert vers la zone 2.

A partir des dispositifs de séparation partent des canalisations munies de pompes, vannes... pour recycler une partie du liquide surnageant et éventuellement une partie des boues vers les moyens pour effectuer la fermentation selon l'étape 1.

Des canalisations amènent le liquide surnageant dans des dispositifs assurant la fermentation anaérobie en écoulement ascendant à travers un lit à garnissage, selon l'étape 3 du procédé. Lesdits dispositifs comprennent une zone 7 de fermentation constituée d'un lit à garnissage reposant sur une grille.

Le garnissage de la zone 7 est composé de préférence d'anneaux ondulés en polychlorure de vinyle du type FLOCOR R ou bien de houppes ou de pompons. D'autres éléments peuvent tout aussi bien convenir : plaques, copeaux... en argile expansée, matière plastique, textile.

Les garnissages des zones 2 et 7 peuvent être identiques sans que cela soit une obligation.

Le dispositif utilisable pour mettre en oeuvre le procédé, est composé d'un seul fermenteur divisé en deux parties, de préférence de volumes sensiblement égaux par une cloison étanche, mais réservant une communication entre les deux parties en sa partie basse, chacune des deux parties est munie d'un garnissage reposant sur une grille couvrant toute la section du fermenteur, comportant en outre au moins une conduite pour l'évacuation des gaz de digestion et
- une canalisation amenant le substrat au-dessus du garnissage de la première partie du fermenteur,
- une canalisation pour la sortie du liquide traité au-dessus du garnissage de la deuxième partie du fermenteur,
- une canalisation de recyclage des boues reliant le fond du fermenteur à la canalisation d'amenée du substrat,
- une canalisation de recyclage du liquide, placée au-dessous du garnissage de la première partie aboutissant à la canalisation d'amenée du substrat.

Afin d'aménager un volume suffisant de la zone de décantation située au-dessous de la grille, ladite grille est placée à une hauteur h (comptée à partir du bas du fermenteur) telle que $h/H = 0,1$ à $0,5$, de préférence $0,25$, H étant la hauteur totale du fermenteur.

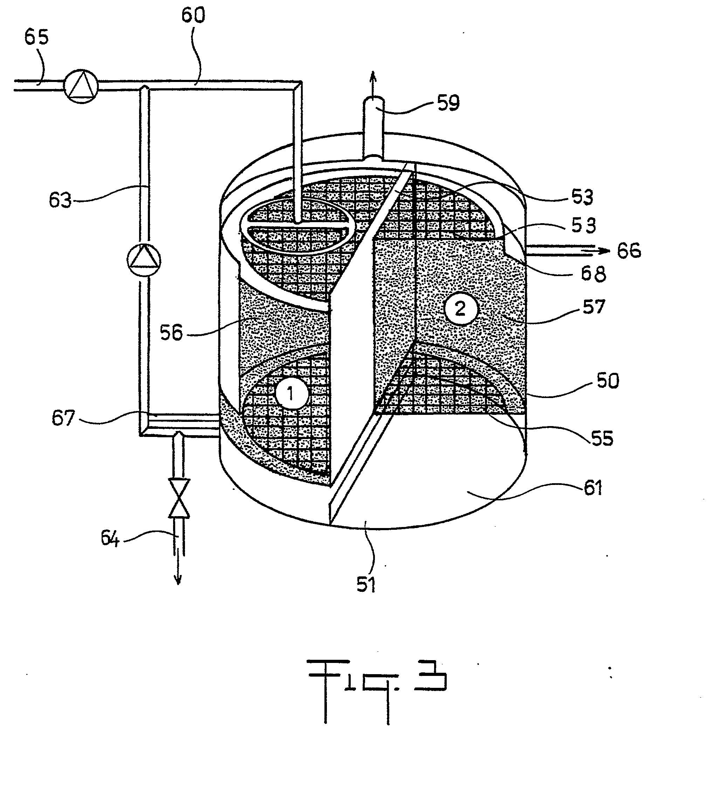

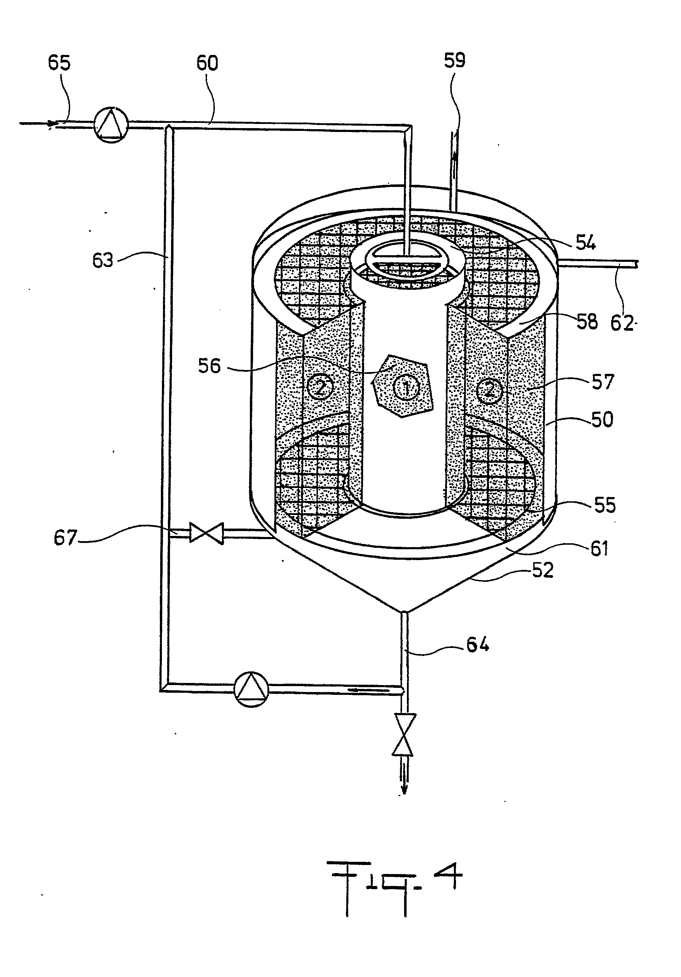

La forme du fond du fermenteur dans laquelle est la zone de séparation peut être variée : conique ou plane par exemple selon les figures 3 et 4, de même, la forme de la cloison : plane ou circulaire par exemple selon les mêmes figures, sans que ces formes soient limitatives.

En se référant aux figures, il va être décrit des dispositifs particuliers avantageusement utilisés pour l'application du procédé.

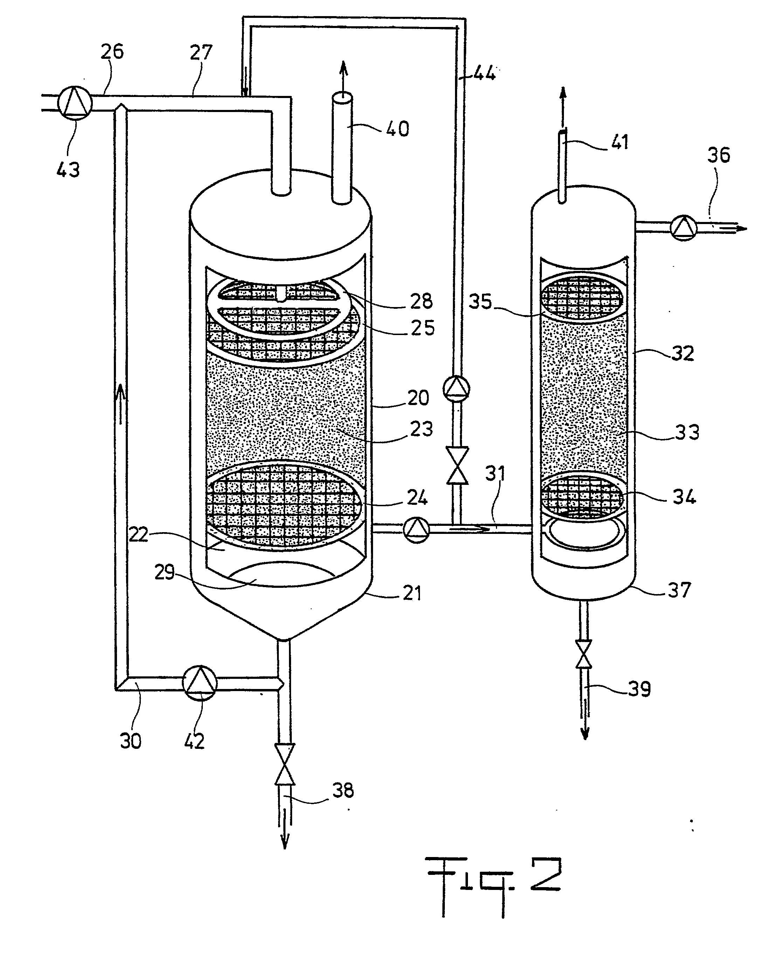

Le dispositif schématisé figure 2 comprend deux fermenteurs 20 et 32 composés d'une virole, d'un toit et d'un fond. Dans l'installation représentée, le fermenteur 20 travaillant avec un écoulement descendant du substrat présente avantageusement un fond conique 21 de façon à aménager dans le bas dudit fermenteur une zone de séparation 22 des MES par décantation. Le fermenteur 20 est muni d'un garnissage 23, placé au-dessus d'une grille 24. De préférence, une grille 25 est placée juste au-dessus des éléments du garnissage, de façon à maintenir fixe ledit garnissage, mais cette disposition n'est par obligatoire.

Ainsi, le substrat 26 est amené par une canalisation 27 au-dessus du garnissage. Des éclateurs ou des bouches d'arrosage 28, placés avantageusement en bout de canalisation 27, assurent une meilleure répartition du substrat sur le garnissage. Le substrat descend alors à travers le garnissage contenant les micro-organismes actifs dans des conditions anaérobies où il subit les réactions de dégradation.

Le mélange sortant se sépare d'une grande partie de ses MES dans la zone de séparation 22 aménagée au-dessous de la grille 24. Des boues 29 sont collectées dans le fond conique 21.

Pour aménager une zone de séparation d'un volume suffisant dans le bas du fermenteur 20, la grille 24 est placée à une hauteur h (comptée à partir du bas du fermenteur 20) telle que $h/H = 0,1$ à $0,5$ et de préférence $h/H = 0,25$ avec H = hauteur totale du fermenteur 20.

De la zone de séparation 22 partent une canalisation 30 pour le recyclage d'une partie des boues 29 vers la canalisation 27 d'alimentation du fermenteur 20 et une canalisation 31 pour le liquide partiellement épuré dans le fermenteur 20 qui l'amène du bas du fermenteur 20 (au-dessous de la grille 24) au bas du fermenteur 32 (au-dessous de la grille 34). Sur cette canalisation est branchée une canalisation 44 pour le recyclage du liquide dans le fermenteur 20. Le fermenteur 32 est aussi muni d'un garnissage 33 reposant sur une grille 34 et éventuellement surmonté d'une grille 35 le maintenant dans une position fixe. Les éléments du garnissage 33 peuvent être les mêmes que ceux du garnissage 23 sans que cela soit obligatoire.

Le liquide arrivant par la canalisation 31 au-dessous de la grille 34 monte dans le garnissage où il se sépare d'une partie de ses MES résiduelles et est encore dégradé par les micro-organismes retenus dans ce garnissage. Il ressort du fermenteur 32 par une canalisation 36 située en haut du fermenteur soit au-dessus du garnissage (tel que représenté sur cette figure 2), soit avec un déversoir situé au niveau du haut du garnissage (tel que représenté sur la figure 3).

Le fermenteur 32 comporte un fond 37 plat, tel que représenté sur la figure 2. En effet, la quantité de MES amenée par le liquide dans la canalisation 31 est relativement faible, de sorte que la collecte des boues dans le bas du fermenteur 32 ne nécessite pas un fond conique.

Dans le cas d'un fermenteur à fond plat fonctionnant en écoulement ascendant avec des substrats peu chargés, il est préférable d'avoir une zone de fermentation la plus importante possible, de sorte que la grille 34 est située (à partir du bas du fermenteur) à une hauteur h telle que $h/H = 0,1$ à $0,25$, H étant la hauteur totale du fermenteur.

Les canalisations 38 et 39 permettent l'évacuation périodique des boues sur chaque fermenteur, les conduites 40 et 41 celle des gaz de digestion. Les débits des fluides sont réglés à l'aide des pompes 42, 43 et de vannes placées sur les canalisations des fluides et ajustés aux valeurs définies par le procédé. Ils seront donc également fonction des sections des fermenteurs 20 et 32.

Afin de réduire les coûts sur les matériels, il est avantageux d'employer pour mettre en oeuvre le procédé un fermenteur unique, par exemple décrit sur les figures 3 et 4.

Ce fermenteur 50 est composé d'une virole, d'un toit et d'un fond plat 51 ou conique 52. Une cloison le sépare en deux parties (1) et (2). C'est une cloison plane 53 sur la figure 3, une cloison cylindrique 54 sur la figure 4. Les cloisons 53 et 54 présentent nécessairement une ouverture dans leur partie basse, de façon que le substrat descendant dans la partie (1) puisse remonter dans la partie (2) après avoir traversé le garnissage de la partie (1) et avoir été en partie décanté.

Cette ouverture peut correspondre à toute la partie au-dessous des grilles 56, 57 - la cloison repose alors sur ces grilles - (figure 4) ou bien une ouverture peut être pratiquée dans la partie de la cloison située juste au-dessous desdites grilles, ouverture suffisante pour laisser passer sans problème le liquide, mais qui retient les boues (figure 3).

Une grille horizontale 55 est solidaire de la cloison 53 ou 54 sur laquelle reposent les garnissage 56, 57. Une grille horizontale 58 est éventuellement placée au-dessus du garnissage pour le maintenir fixe.

Dans les deux cas, figures 3 et 4, la grille 55 est placée à une hauteur h (comptée à partir du bas du fermenteur) telle que $h/H = 0,1$ à 0,5 et de préférence $h/H = 0,25$ avec H = hauteur totale du fermenteur 50 de façon à aménager au-dessous de ladite grille 55 une zone de décantation 61 suffisante.

Une canalisation 60 amène le substrat à traiter dans la partie (1) au-dessus du garnissage 56. Le substrat se compose de l'effluent à traiter amené par la conduite 65, du liquide à recycler amené par la conduite 67 et des boues de recyclage amenées par la conduite 63. Le substrat traverse le garnissage en écoulement descendant, se sépare d'une grande partie des MES dans la zone de décantation 61 avant de remonter dans le garnissage 57 de la partie (2) d'où il sort épuré par la canalisation 62 située soit au-dessus du garnissage 57 (figure 4), soit par un déversoir 66 situé dans le haut du garnissage 57 (figure 3).

La conduite de recyclage 63 réintroduit dans la conduite 60 une partie des boues décantées, une autre partie étant évacuée périodiquement par la conduite 64 pour éviter l'engorgement du fermenteur.

Les garnissages 56, 57 sont identiques ou différents. Il est préféré des éléments anneaux FLOCOR R en polychlorure de vinyle déposés en vrac de façon non ordonnée ou bien un ensemble de houppes et de pompons suspendus dans le fermenteur. Les débits sont ajustés par des pompes et des vannes, de façon à respecter les conditions définies par le procédé.

Il a été décrit dans ces figures des dispositifs particulièrement avantageux pour mettre en oeuvre le procédé, mais tout autre dispositif fonctionnant selon les caractéristiques du procédé objet principal de l'invention serait dépendant de l'invention.

L'exemple suivant permettra d'illustrer l'invention.

Un fermenteur de volume utile 10 m³ du type de celui représenté figure 3 de section 2,5 m² et hauteur 4 m est séparé en deux parties de volumes égaux par une mince cloison plane. Chacune des parties est remplie de FLOCOR R.

La partie (1) est alimentée par un substrat composé de :
- l'effluent à traiter : une vinasse de canne avec DCO = 50 000 mg/l
MES = 3 g/l
sels = 13,6 g/l Extrait sec = 65 g/l
à un débit de 0,4 m³/h,
- le liquide est recyclé à un débit de 1,25 m³/h et les boues à 0,15 m³/h,
- dans la partie (1) le substrat s'écoule vers le bas avec une vitesse de 1,44 m/h et dans la partie (2) la vitesse ascensionnelle est de 0,32 m/h,
on obtient
- un substrat traité dans la partie (1) avec une DCO de 15 g/l,
- un substrat sortant de la partie (2) avec une DCO de 5 g/l et 0,3 g/l de MES,
- une production de gaz ($CH_4 + CO_2$) de 12,6 m³ de gaz/m³ fermenteur.jour.

## Revendications

1. Procédé de traitement biologique d'effluents, contenant des matières organiques en suspension et/ou en solution présentant une $DCD \geq 5000$ mg/l et une teneur en MES $\geq 1$ g/l par fermentation méthanique comprenant deux étapes successives de fermentation anaérobie à travers un lit à garnissage, la première en écoulement descendant, la seconde en écoulement ascendant, ces deux opérations étant séparées par une étape de décantation suffisante pour donner naissance à un liquide surnageant et à des boues caractérisé en ce que l'on recycle ledit liquide surnageant vers ladite première étape avec un débit supérieur à celui de l'effluent à traiter.

2. Procédé selon la revendication 1, caractérisé en ce que l'on réalise également le recyclage d'une partie desdites boues et que dans ce cas le débit du liquide surnageant représente au moins 90% environ du débit total de recyclage.

3. Procédé selon l'une des revendications 1 ou 2, caractérisé en ce que le garnissage est composé d'anneaux en polychlorure de vinyle.

4. Procédé selon l'une des revendications 1 ou 2, caractérisé en ce que le garnissage est composé de houppes ou de pompons.

5. Procédé selon l'une des revendications 1 à 4, caractérisé en ce que le débit du recyclage est compris entre 1 et 10 fois celui de l'effluent à traiter.

## Claims

1. Process for the biological treatment of effluents containing organic matters in suspension and/or in solution, having a COD $\geq 5000$ mg/l and an MIS content $\geq 1$ g/l by methane fermentation, comprising two successive steps of anaerobic fermentation through a packing bed, the first step in downward flow, the second in upward flow, said two operations are separated by a decantation step sufficient to give rise to a supernatant liquid and to sludges, characterized in that said supernatant liquid is recycled toward said first step with a flow rate higher than than of the effluent to be treated.

2. Process according to claim 1, characterized in that a part of said sludges is also recycled and in that the flow rate of the supernatant liquid represents at least 90% of the total recycling flow.

3. Process according to any one of claims 1 or 2, characterized in that the packing is composed of rings in polyvinyl chloride.

4. Process according to one of claims 1 or 2, characterized in that the packing is composed of tufts and pompons.

5. Process according to one of claims 1 to 4, characterized in that the recycling flow rate is between one and ten times that of the effluent to be treated.

**Patentansprüche**

1. Verfahren zur biologischen Aufbereitung von organische Stoffe in Suspension und/oder Lösung enthaltenden Abwässern mit einem chemischen Sauerstoffbedarf (CSB) von ≥ 5000 mg/l und einem Gehalt an Schwebstoffen von ≥ 1 g/l mittels Methangärung, umfassend zwei aufeinanderfolgende anaerobe Gärstufen durch ein Schüttbett, wobei die erste im fallenden Fluß und die zweite im steigenden Fluß erfolgt und die beiden Stufen durch eine Dekantierstufe getrennt sind, welche zur Bildung eines Überstandes und von Schlämmen ausreicht, dadurch gekennzeichnet, daß der Überstand in einer Menge zur ersten Stufe rückgeführt wird, die höher als jene des zu behandelnden Abwassers ist.

2. Verfahren nach Anspruch 1, dadurch gekennzeichnet, daß auch ein Teil der Schlämme rückgeführt wird und in diesem Fall die Menge an Überstand mindestens etwa 90% der gesamten Rückführungsmenge ausmacht.

3. Verfahren nach einem der Ansprüche 1 oder 2, dadurch gekennzeichnet, daß die Schüttschicht durch Vinylpolychloridringe gebildet ist.

4. Verfahren nach einem der Ansprüche 1 oder 2, dadurch gekennzeichnet, daß die Schüttschicht durch Flockenbündel oder Schleifen gebildet ist.

5. Verfahren nach einem der Ansprüche 1 bis 4, dadurch gekennzeichnet, daß die Rückführungsmenge das 1- bis 10fache jener des zu behandelnden Abwassers ausmacht.

Fig. 1

Fig. 2

Fig. 3

Fig. 4